(19) **Europäisches Patentamt / European Patent Office / Office européen des brevets**

(11) **EP 3 054 281 B1**

(12) **FASCICULE DE BREVET EUROPEEN**

(45) Date de publication et mention
de la délivrance du brevet:
**29.07.2020 Bulletin 2020/31**

(51) Int Cl.:
*G01N 21/47* (2006.01)   *G01N 21/64* (2006.01)
*A61B 5/00* (2006.01)   *G01N 21/65* (2006.01)

(21) Numéro de dépôt: **16154145.3**

(22) Date de dépôt: **03.02.2016**

(54) **DISPOSITIF DE MESURE D'UN SIGNAL OPTIQUE RÉTRODIFFUSÉ PAR UN ÉCHANTILLON**

VORRICHTUNG ZUM MESSEN EINES VON EINER PROBE RÜCKGESTREUTEN OPTISCHEN SIGNALS

DEVICE FOR MEASURING AN OPTICAL SIGNAL BACKSCATTERED BY A SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.02.2015 FR 1550981**

(43) Date de publication de la demande:
**10.08.2016 Bulletin 2016/32**

(73) Titulaire: **Commissariat à l'Énergie Atomique et aux Énergies Alternatives
75015 Paris (FR)**

(72) Inventeurs:
- **ROIG, Blandine
  56160 Guémené-sur-Scorff (FR)**
- **KOENIG, Anne
  38410 Saint Martin d'Uriage (FR)**
- **DINTEN, Jean-Marc
  69008 Lyon (FR)**
- **PERRAUT, François
  38134 Saint Joseph de Rivière (FR)**

(74) Mandataire: **GIE Innovation Competence Group
310, avenue Berthelot
69372 Lyon Cedex 08 (FR)**

(56) Documents cités:
**EP-A2- 1 262 763       EP-B1- 1 828 753
WO-A1-2010/054203    US-A- 290 275
US-A1- 2011 178 379**

- **ANNE KOENIG ET AL: "Diffuse reflectance spectroscopy: a clinical study of tuberculin skin tests reading", PROCEEDINGS OF SPIE, vol. 8592, 21 février 2013 (2013-02-21), pages 85920S-85920S-8, XP055090850, ISSN: 0277-786X, DOI: 10.1117/12.2002314**
- **THUELER P ET AL: "In vivo endoscopic tissue diagnostics based on spectroscopic absorption, scattering, and phase function properties", JOURNAL OF BIOMEDICAL OPTICS, vol. 8, no. 3, juillet 2003 (2003-07), pages 495-503, XP055282637,**

## Description

### Domaine technique

L'invention se situe dans le domaine de l'instrumentation optique destinée à caractériser des échantillons, et notamment des échantillons biologiques, et plus particulièrement la peau.

### Description de l'art antérieur

Les mesures optiques permettant de caractériser des échantillons sont répandues. Il s'agit en particulier de caractériser les propriétés optiques, ou la nature des matériaux qui composent un échantillon. On peut notamment citer les mesures basées sur la détection d'un signal rétrodiffusé par un échantillon illuminé par un faisceau lumineux. Il s'agit en particulier de la spectrométrie Raman, l'imagerie de fluorescence ou la spectrométrie de réflectance diffuse.

La demande de brevet WO2010/054203 décrit un dispositif permettant une estimation de propriétés optiques d'un échantillon par une mesure sans contact. Il en est de même du document EP1262763. Dans ces deux documents, une lentille relais est disposée entre des fibres optiques et l'échantillon, les fibres optiques permettant l'illumination d'un échantillon ainsi que la détection d'un signal rétrodiffusé par l'échantillon. Le document EP1828753 décrit un dispositif pour réaliser une mesure par spectrométrie Raman en profondeur dans un échantillon. La profondeur de mesure peut être modulée en ajustant une distance entre une fibre destinée à illuminer l'échantillon et une fibre destinée à détecter un signal Raman rétrodiffusé par l'échantillon. Le document US2011/0178379 décrit un dispositif de spectrométrie Raman destiné à être appliqué à distance d'un échantillon à caractériser.

La spectrométrie de réflectance diffuse, souvent désignée par l'acronyme anglais DRS (Diffuse Reflectance Spectroscopy), consiste à exploiter la lumière rétrodiffusée par un objet diffusant soumis à un éclairement, généralement ponctuel. Cette technique s'avère performante pour caractériser des propriétés optiques d'objets, en particulier les propriétés de diffusion ou d'absorption.

Mise en œuvre sur la peau, cette technique permet par exemple de caractériser le derme, comme décrit dans les documents Thueler P "In vivo endoscopie tissue diagnostics based on spectroscopic absorption, scattering and phase function properties" ou dans la publication Koenig A "Diffuse reflectance spectroscopy : a clinical study of tuberculin skin tests reading", ou encore dans le document EP 2762064. Les auteurs de ce document décrivent une sonde de mesure destinée à être appliquée contre la peau. Cette sonde comporte une fibre optique centrale, dite fibre d'excitation, destinée à diriger un faisceau lumineux vers un échantillon de peau. Des fibres optiques, disposées autour de la fibre centrale, dites fibres de détection, recueillent un signal optique rétrodiffusé par le derme. Des moyens d'analyse spectrale du signal optique ainsi collecté, couplés à des algorithmes de calcul, permettent d'estimer des paramètres du derme, en particulier la concentration de certains chromophores, par exemple l'oxyhémoglobine ou la déoxyhémoglobine, mais aussi des paramètres gouvernant le parcours des photons dans le derme, notamment le coefficient de diffusion réduit $\mu s'$ ainsi que le coefficient d'absorption $\mu a$.

Selon les principes de la diffusion de la lumière dans un milieu diffusant, plus l'écartement entre la fibre d'excitation et une fibre de détection est important, plus la lumière recueillie par la fibre de détection est fonction des propriétés optiques dans les couches profondes du derme, c'est-à-dire au-delà d'une plage de profondeur comprise entre 300 $\mu m$ et quelques mm. Autrement dit, plus la distance entre la fibre d'excitation et la fibre de détection augmente, plus le résultat est représentatif des propriétés optiques des couches profondes du milieu examiné. A l'inverse, plus cette distance diminue, plus le résultat est représentatif des couches superficielles du milieu diffusant.

Or, afin d'effectuer une mesure suffisamment sensible, chaque fibre a un diamètre égal à quelques centaines de microns, typiquement 500 $\mu m$ pour la fibre d'excitation et 300 $\mu m$ pour chaque fibre de détection. Par ailleurs, du fait de contraintes de fabrication, il est difficilement envisageable de réduire significativement la distance entre ces fibres. Il en résulte que l'écartement minimal entre chaque fibre ne peut pas être inférieur à une valeur seuil, généralement de l'ordre de quelques centaines de $\mu m$, généralement au-delà de 300 $\mu m$.

De ce fait, alors que les dispositifs décrits dans les publications Thueler, Koenig et EP 2762064 sont adaptés à la caractérisation du derme, ils ne sont pas aptes à caractériser l'épiderme, ce dernier s'étendant, selon les individus et les zones corporelles, sur les 100 à 300 premiers microns de la peau.

L'invention vise à résoudre ce problème, en proposant un dispositif de mesure apte à caractériser l'épiderme et, de façon plus générale, les propriétés optiques de la couche superficielle d'un milieu diffusant, tout en conservant des dimensions comparables au dispositif de l'art antérieur.

### Description de l'invention

Un objet de l'invention est un dispositif de mesure d'un signal optique produit par un échantillon comportant une source de lumière apte à émettre un faisceau lumineux vers une surface dudit échantillon, de façon à former, sur ladite surface, une zone élémentaire d'illumination, au moins une fibre optique de détection, s'étendant entre une extrémité proximale, apte à être couplée à un photodétecteur, et une extrémité distale, apte à collecter un signal optique rétrodiffusé par ledit échantillon lorsqu'il est exposé audit faisceau lumineux, un système optique, présentant un facteur de grandissement et un axe optique, ledit système optique étant apte à conjuguer l'extrémité distale de chaque fibre optique de détection à une zone élémentaire de détection située sur la surface de l'échantillon, de telle sorte que la distance, dite distance de rétrodiffusion, entre la zone élémentaire d'illumination et chaque zone élémentaire de détection, perpendiculairement audit axe optique, est fonction dudit facteur de grandissement. Selon l'invention, le système optique est configuré de telle sorte qu'au moins une zone élémentaire de détection est séparée de ladite zone élémentaire d'illumination, la distance de rétrodiffusion séparant ladite zone élémentaire de détection de ladite zone élémentaire d'illumination étant inférieure à 200 μm.

Ainsi, de préférence, chaque zone élémentaire de détection est distincte de la zone élémentaire d'illumination et ne se superpose pas à cette dernière. Elle est séparée de cette dernière par une distance non nulle.

Selon un mode de réalisation, le système optique est apte à conjuguer ladite source de lumière à ladite zone élémentaire d'illumination située sur la surface de l'échantillon,

Ainsi, si la distance entre le faisceau lumineux et l'extrémité distale d'une fibre optique de détection est, en amont du système optique, égale à une première distance, la distance entre la zone élémentaire d'illumination et la zone élémentaire de détection, dite distance de rétrodiffusion, est, à la surface de l'échantillon, égale à ladite première distance pondérée par ledit facteur de grandissement.

Le dispositif peut comporter :

- une première fibre optique de détection, dont l'extrémité distale est située à une première distance dudit faisceau lumineux,
- une deuxième fibre optique de détection, dont l'extrémité distale est située à une deuxième distance dudit faisceau lumineux, ladite deuxième distance étant supérieure à ladite première distance,

de façon à constituer, à la surface de l'échantillon,

- une première zone élémentaire de détection, conjuguée à ladite première fibre optique de détection, située à une première distance de rétrodiffusion de ladite zone d'illumination, fonction de ladite première distance et du facteur de grandissement,
- une deuxième zone élémentaire de détection, conjuguée à ladite deuxième fibre optique de détection, située à une deuxième distance de rétrodiffusion de ladite zone d'illumination, fonction de ladite deuxième distance et du facteur de grandissement.

Le dispositif peut également comporter :

- un premier groupe de premières fibres optiques de détection, dont l'extrémité distale est située à une première distance dudit faisceau lumineux,

- un deuxième groupe de deuxièmes fibres optiques de détection, dont l'extrémité distale est située à une deuxième distance dudit faisceau lumineux, ladite deuxième distance étant supérieure à ladite première distance,

de façon à constituer, à la surface de l'échantillon,

- une premier groupe de premières zones élémentaires de détection, chaque première zone élémentaire de détection étant conjuguée à l'extrémité distale d'une fibre optique de détection du premier groupe, la distance entre chaque première zone élémentaire de détection et la zone élémentaire d'illumination étant fonction de ladite première distance et du facteur de grandissement dudit système optique,
- une deuxième groupe de deuxièmes zones élémentaires de détection, chaque deuxième zone élémentaire de détection étant conjuguée à l'extrémité distale d'une fibre optique de détection du deuxième groupe, la distance entre chaque deuxième zone élémentaire de détection et la zone élémentaire d'illumination étant fonction de ladite deuxième distance et du facteur de grandissement dudit système optique.

Selon un mode de réalisation,

- les extrémités distales desdites premières fibres de détection sont coplanaires et réparties autour dudit faisceau lumineux, le long d'un cercle de rayon égal à ladite première distance, centré sur ledit faisceau lumineux,
- les extrémités distales desdites deuxièmes fibres de détection sont coplanaires et réparties autour dudit faisceau lumineux, le long d'un cercle de rayon égal à la deuxième distance, centré sur ledit faisceau lumineux.

La source de lumière peut comporter une fibre optique d'illumination, s'étendant entre

- une extrémité proximale, apte à recevoir ladite lumière,
- et une extrémité distale, apte à émettre un faisceau lumineux, notamment en direction de la surface de l'échantillon.

La source de lumière peut comporter un système de renvoi, apte à réfléchir, en direction de l'échantillon, ledit faisceau lumineux. Le système de renvoi est par exemple une lame semi-réfléchissante. Dans ce cas, le dispositif peut comporter une fibre de détection apte à collecter un signal optique réfléchi ou rétrodiffusé à 180° par l'échantillon.

La source de lumière peut être choisie parmi une source de lumière blanche, une source Laser, une diode électroluminescente.

De préférence, ladite source de lumière est apte à diriger ledit faisceau lumineux parallèlement audit axe optique.

Le dispositif peut comporter un contrôleur de la distance entre ledit système optique et ladite surface de l'échantillon.

Selon un mode de réalisation, le contrôleur de distance comporte :

- un analyseur de l'intensité lumineuse du signal optique collecté par une fibre optique de détection,
- ou une caméra couplée à l'extrémité proximale d'au moins une fibre optique de détection.

Le contrôleur de distance peut comporter un support, s'étendant selon l'axe optique du système optique, et apte à être appliqué contre la surface de l'échantillon.

Dans ce cas, le dispositif peut comporter une fenêtre transparente, solidaire dudit support, s'étendant perpendiculairement audit axe optique, la fenêtre étant apte à être appliquée contre la surface de l'échantillon, de telle sorte que cette dernière épouse la forme de la fenêtre.

Le dispositif peut comporter un photodétecteur choisi parmi une photodiode, un spectrophotomètre, notamment un spectromètre Raman ou un détecteur d'un signal fluorescent.

Un autre objet de l'invention est un procédé de détermination d'une propriété optique ($\mu_a$, $\mu_s$, $\mu_s'$, g) d'un échantillon, à l'aide d'un dispositif selon l'invention, comportant les étapes suivantes :

- illumination d'une surface de l'échantillon à l'aide d'un faisceau lumineux, de façon à constituer, à la surface dudit échantillon, une zone élémentaire d'illumination, correspondant à la partie de ladite surface éclairée par ledit faisceau,
- détection de N signaux optiques, rétrodiffusés par l'échantillon, chaque signal optique émanant de la surface de l'échantillon à une distance, dite distance de rétrodiffusion, de ladite zone élémentaire d'illumination, N étant un entier supérieur ou égal à 1,
- détermination d'au moins une propriété optique de l'échantillon, par comparaison entre :

  - une fonction de chaque signal ainsi détecté,
  - et une pluralité d'estimations de ladite chaque estimation étant réalisée en considérant une valeur prédéterminée de ladite propriété optique.

La propriété optique à déterminer peut être un facteur d'anisotropie, représentant un angle moyen de diffusion, un coefficient de diffusion réduit, un coefficient de diffusion ou un coefficient d'absorption.

Le procédé peut comprendre :

- la détection d'au moins deux signaux optiques rétrodiffusés émanant de la surface de l'échantillon,

  - un signal optique rétrodiffusé proche, émis à une distance de rétrodiffusion inférieure à 200 $\mu$m,
  - un signal optique rétrodiffusé éloigné, émis à une distance de rétrodiffusion supérieure à

  celle correspondant audit signal rétrodiffusé proche, et par exemple supérieure à 200 $\mu$m,
- la détermination d'une première propriété optique à partir du signal proche,
- la détermination d'une deuxième propriété optique, différente de la première propriété optique, à partir du signal éloigné.

Il peut également comprendre :

- la détection d'au moins deux signaux optiques rétrodiffusés proches, à deux distances de rétrodiffusion différentes, les deux distances étant inférieures à 200 $\mu$m,

- la détermination d'un coefficient de diffusion réduit à partir du signal optique éloigné,
- puis la détermination d'un coefficient de diffusion et d'un coefficient d'anisotropie à partir des deux signaux optiques rétrodiffusés proches, en utilisant le coefficient de diffusion réduit préalablement déterminé.

L'échantillon examiné peut être la peau d'un humain ou d'un animal.

L'intensité du signal optique rétrodiffusé peut être mesurée à une pluralité de longueurs d'ondes.

## Description des figures.

Les figures 1 et 2 représentent un premier mode de réalisation d'un dispositif selon l'invention.

Les figures 3A et 3B représentent respectivement un schéma du dispositif selon l'art antérieur et selon l'invention.

La figure 4 est un schéma représentant le parcours de la lumière dans un échantillon diffusant en fonction de la distance de rétrodiffusion.

Les figures 5A et 5B représentent des variantes du premier mode de réalisation d'un dispositif selon l'invention.

La figure 6 représente un deuxième mode de réalisation d'un dispositif selon l'invention.

Les figures 7A, 7B et 7C représentent les variantes d'un troisième mode de réalisation d'un dispositif selon l'invention.

La figure 8 représente un quatrième mode de réalisation d'un dispositif selon l'invention

Les figures 9A à 9C représentent les étapes de procédé de détermination des propriétés optiques d'un échantillon, pouvant être mises en œuvre à l'aide d'un dispositif selon l'invention.

Les figures 10A et 10B représentent les résultats d'une modélisation de la réflectance pour deux distances de rétrodiffusion respectives, la réflectance étant estimée pour différents couples de valeurs du coefficient de d'absorption $\mu a$ et du coefficient de diffusion réduit $\mu s'$.

## Description détaillée

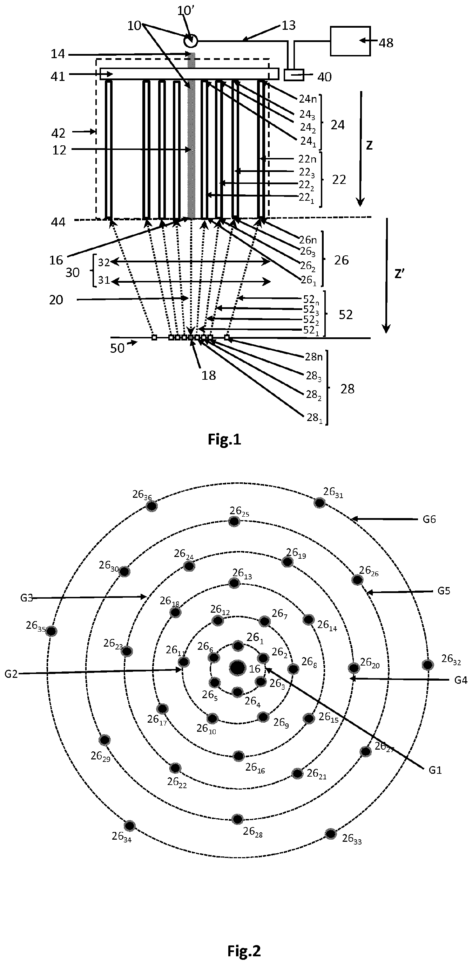

La figure 1 représente un premier mode de réalisation d'un dispositif selon l'invention. Il comporte une source de lumière 10, qui comprend dans ce premier exemple une source de lumière blanche 10'.

La source de lumière 10 comprend, dans ce premier mode de réalisation, une fibre optique d'illumination 12, s'étendant entre une extrémité proximale 14 et une extrémité distale 16. La fibre optique d'illumination 12 est apte à collecter la lumière, par une extrémité proximale 14 et à émettre un faisceau lumineux 20 vers l'échantillon par une extrémité distale 16, ledit faisceau lumineux étant alors dirigé vers la surface d'un échantillon 50. Dans une telle configuration, la source de lumière 10 est dite fibrée.

Le diamètre de la fibre optique d'émission 12 est compris entre 100 $\mu$m et 1 mm, et est par exemple égal à 400 $\mu$m.

Par source ponctuelle, on entend une source dont l'aire est inférieure à 1 cm$^2$, et de préférence inférieure à 5 mm$^2$, et encore de préférence inférieure à 1 mm$^2$.

Le dispositif comporte également une pluralité de fibres optiques de détection $22_1$, $22_2$, $22_3$...$22_n$, l'indice n étant compris entre 1 et N, N désignant le nombre de fibres optiques de détection dans le dispositif. N est un entier naturel compris généralement compris entre 1 et 100, et préférentiellement compris entre 5 et 50. Chaque fibre de détection $22_1$, $22_2$, $22_3$...$22_n$ s'étend entre une extrémité proximale $24_i$, $24_2$, $24_3$...$24_n$ et une extrémité distale $26_1$, $26_2$, $26_3$...$26_n$.

Sur la figure 1, les références 22, 24 et 26 désignent respectivement l'ensemble des fibres de détection, l'ensemble des extrémités proximales des fibres de détection et l'ensemble des extrémités distales des fibres de détection.

Le diamètre de chaque fibre optique de détection 22 est compris entre 50 $\mu$m et 1 mm, et est par exemple égal à 300 $\mu$m.

L'extrémité proximale 24 de chaque fibre optique de détection 22 est apte à être optiquement couplée à un photodétecteur 40.

L'extrémité distale $26_1$, $26_2$, $26_3$...$26_n$ de chaque fibre optique de détection 22 est apte à collecter respectivement un signal optique $52_1$, $52_2$, $52_3$...$52_n$ rétrodiffusé par l'échantillon 50, lorsque ce dernier est exposé au faisceau lumineux 20.

Le photodétecteur 40 est apte à détecter chaque signal optique $52_1$, $52_2$, $52_3$...$52_n$ de façon à former un signal $S_1$, $S_2$, $S_3$, ...$S_n$ respectivement représentatif de chaque signal optique détecté.

Il peut s'agir d'un spectrophotomètre, apte à établir le spectre en longueur d'onde du signal optique collecté par la fibre optique de détection 22 auquel il est couplé.

Le photodétecteur est apte à être connecté à un microprocesseur 48, ce dernier étant configuré pour mettre en œuvre les procédés décrits ci-après.

Les fibres optiques de détection 22 s'étendent parallèlement les unes aux autres, parallèlement à un axe longitudinal autour de la fibre optique d'émission 12. Elles sont maintenues fixes les unes par rapport aux autres par un élément de maintien 42. Leurs extrémités distales 26 sont coplanaires, et définissent un plan de détection 44.

Une fibre optique 13, dite fibre de retour d'excitation, relie la source de lumière 10 au photodétecteur 4. Cette fibre optique est utile pour réaliser une mesure de calibration détaillée ultérieurement. Cette fibre optique de retour d'excitation n'est pas représentée sur les figures suivantes, mais peut être présente sur l'ensemble des modes de réalisation.

La figure 2 représente une vue en coupe du dispositif, dans le plan de détection, formé par l'ensemble des extrémités distales 26 des N fibres de détection. Dans cet exemple, N est égal à 36. Comme on peut le voir, les fibres optiques de détection sont réparties selon :

- un premier groupe $G_1$ de six fibres optiques de détection $22_1$...$22_6$, disposées régulièrement le long d'un cercle centré sur la fibre optique d'émission 12, de telle sorte que l'extrémité distale $26_1$...$26_6$ de chaque fibre de ce groupe soit distante de l'extrémité distale 16 de la fibre optique d'émission 12 d'une première distance $d_1$ égale à 300 $\mu$m.

- un deuxième groupe $G_2$ de six fibres optiques de détection $22_7$...$22_{12}$, disposées régulièrement le long d'un cercle centré sur la fibre optique d'émission 12, de telle sorte que l'extrémité distale $26_7$...$26_{12}$ de chaque fibre de ce groupe soit distante de l'extrémité distale 16 de la fibre optique d'émission 12 d'une deuxième distance $d_2$ égale à 700 $\mu$m.

- un troisième groupe $G_3$ de six fibres optiques de détection $22_{13}$...$22_{18}$, disposées régulièrement le long d'un cercle centré sur la fibre optique d'émission 12, de telle sorte que l'extrémité distale $26_{13}$...$26_{18}$ de chaque fibre de ce groupe soit distante de l'extrémité distale 16 de la fibre optique d'émission 12 d'une troisième distance $d_3$ égale à 1,1 mm.

- un quatrième groupe $G_4$ de six fibres optiques de détection $22_{19}$...$22_{24}$, disposées régulièrement le long d'un cercle centré sur la fibre optique d'émission 12, de telle sorte que l'extrémité distale $26_{19}$...$26_{24}$ de chaque fibre de ce groupe soit distante de l'extrémité distale 16 de la fibre optique d'émission 12 d'une quatrième distance $d_4$ égale à 1,5 mm.

- un cinquième groupe $G_5$ de six fibres optiques de détection $22_{25}$...$22_{30}$, disposées régulièrement le long d'un cercle centré sur la fibre optique d'émission 12, de telle sorte que l'extrémité distale $26_{25}$...$26_{30}$ de chaque fibre de ce groupe soit distante de l'extrémité distale 16 de la fibre optique d'émission 12 d'une cinquième distance $d_5$ égale à 2 mm.

- un sixième groupe $G_6$ de six fibres optiques de détection $22_{31}$...$22_{36}$, disposées régulièrement le long d'un cercle centré sur la fibre optique d'émission 12, de telle sorte que l'extrémité distale $26_{31}$...$26_{36}$ de chaque fibre de ce groupe soit distante de l'extrémité distale 16 de la fibre optique d'émission 12 d'une sixième distance $d_6$ égale à 2,5 $\mu$m.

Lorsqu'on évoque une distance entre deux fibres, ou entre une fibre ou un faisceau lumineux, on entend une distance centre à centre.

Ainsi, chaque extrémité distale $26_n$ d'une fibre optique de détection $22_n$ est placée, dans un plan perpendiculaire à l'axe longitudinal Z selon lequel s'étendent ces fibres, à une distance $d_n$ de la source de lumière 10 (c'est-à-dire de l'extrémité distale 16 de la fibre d'émission 12), et, par conséquent, à une distance $d_n$ du faisceau lumineux 20 dirigé vers l'échantillon 50.

Le dispositif peut comporter un photodétecteur 40, apte à être couplé à l'extrémité proximale 24n de chaque fibre optique de détection 22n. Dans cet exemple, le photodétecteur est un spectrophotomètre, apte à déterminer le spectre d'un signal optique $52_1$...$52_n$ rétrodiffusé par l'échantillon lorsque ce dernier est exposé au faisceau lumineux 20. Pour cela, les extrémités proximales 24 de chaque groupe de fibres optiques de détection, décrits ci-dessus, sont regroupées et sont, groupe par groupe, successivement couplées au photodétecteur 40 au moyen d'un commutateur optique 41. Ainsi, le photodétecteur permet de mesurer le rayonnement rétrodiffusé 52 par l'échantillon, sous l'effet d'une illumination par le faisceau lumineux 20.

Le dispositif comporte également un système optique 30, présentant un facteur de grandissement G et un axe optique Z'. Dans cet exemple, l'axe optique Z' est confondu avec l'axe longitudinal Z selon lequel s'étendent les fibres optiques de détection, ce qui constitue une configuration préférée. Dans cet exemple, le système optique 30 comporte:

- un objectif de microscope 31, placé en configuration foyer infini, apte à produire une image à l'infini de la surface de l'échantillon observé, lorsque ce dernier est placé au foyer de cet objectif. Dans cet exemple, il s'agit d'un objectif de marque Zeiss, de référence Plan Aprochromat 20X, d'ouverture numérique 0.8, et de distance focale $f_1 = 8,25$ mm,

- un doublet achromat 32, de distance focale $f_2 = 50$ mm, accolé à l'objectif 31, apte à projeter l'image fournie par ce dernier dans le plan de détection 44, ce plan étant placé à la distance focale du doublet.

Le système optique 30 décrit ci-dessus présente un facteur de grandissement G égal au rapport des distances focales, soit $G \approx 6$.

De préférence, le système optique 30 est amovible et interchangeable, ce qui permet, en utilisant le même dispositif, l'utilisation de systèmes optiques présentant des facteurs de grandissements différents.

D'une façon générale, le système optique 30 permet de former une image de la surface de l'échantillon 50 sur un plan de détection 44 formé par les extrémités distales 26 de chaque fibre optique de détection 22, avec un facteur de grandissement G donné. Ainsi, chaque extrémité distale $26_1$, $26_2$...$26_n$ est respectivement conjuguée à une zone élémentaire de détection $28_1$, $28_2$...$28_n$ de la surface de l'échantillon. De cette façon, chaque fibre optique de détection $22_1$, $22_2$,...$22_n$ est apte à collecter respectivement un signal optique élémentaire $52_1$, $52_2$...$52_n$ rétrodiffusé par l'échantillon, chaque signal optique élémentaire $52_1$, $52_2$...$52_n$ émanant de la zone élémentaire de détection $28_1$, $28_2$...$28_n$.

Ainsi, quel que soit le mode de réalisation, chacune desdites extrémités distales $26_1$, $26_2$...$26_n$ peut être située dans un plan focal image du système optique 30, et conjuguée à une zone élémentaire de détection $28_1$, $28_2$...$28_n$ située dans le plan focal objet dudit système optique, à la surface de l'échantillon.

De même, l'extrémité distale 16 de la fibre d'émission 12 est conjuguée à une zone élémentaire d'illumination 18 à la surface de l'échantillon, cette zone élémentaire d'illumination constituant le point d'impact du faisceau lumineux 20 à la surface de l'échantillon 50.

D'une façon générale, et quelle que soit le mode de réalisation, le terme zone élémentaire désigne une partie de la surface de l'échantillon dont les dimensions sont suffisamment faibles pour considérer qu'elle est traversée par un rayonnement lumineux homogène. Autrement dit, une zone élémentaire est une zone de forme délimitée, de préférence ponctuelle, c'est-à-dire dont le diamètre ou la diagonale est inférieure à 5 mm, et de préférence inférieure à 1 mm, voire inférieure à 500 $\mu$m.

Une zone élémentaire d'illumination 18 est traversée par le faisceau lumineux 20, se propageant en direction de l'échantillon 50, tandis qu'une zone élémentaire de détection $28_n$ est traversée par un rayonnement rétrodiffusé (ou signal optique) $52_n$, ce rayonnement étant produit par la rétrodiffusion, dans l'échantillon, du faisceau lumineux 20. Le couplage optique, réalisé par le système optique 30, permet à chaque fibre de détection $22_n$ de collecter le rayonnement rétrodiffusé élémentaire $52_n$, ce dernier correspondant au rayonnement rétrodiffusé traversant la zone élémentaire 28n.

L'élément de maintien 42 peut assurer une liaison rigide entre les fibres optiques de détection 22 et le système optique 30, de façon à maintenir le plan de détection 44, formé par les extrémités distales 26 des fibres optiques de détection à une distance fixe du système optique 30.

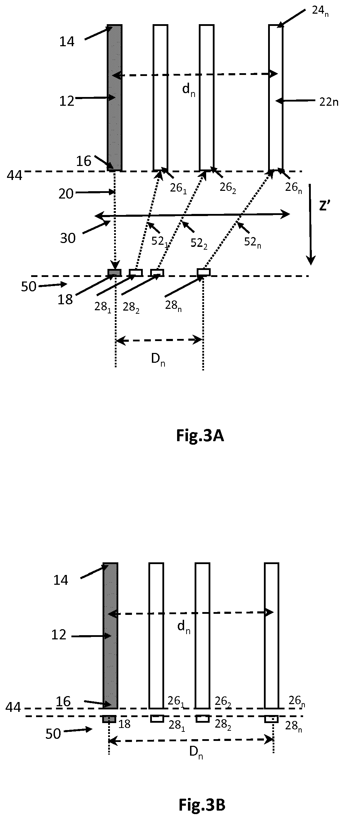

En lien avec la figure 3A, et cela constitue un des points essentiels de l'invention, si $d_n$ est la distance entre l'extrémité distale 26n d'une fibre de détection 22n et l'extrémité distale 16 de la fibre d'émission 12, distance calculée dans un plan perpendiculaire à l'axe optique Z', la distance Dn entre la zone élémentaire de détection 28n, conjuguée de ladite extrémité distale 26n, et la zone élémentaire d'illumination 18, conjuguée de ladite extrémité distale 16, est telle que :

$$Dn = \frac{dn}{G}$$

La distance $D_n$ est appelée distance de rétrodiffusion, car elle correspond à la distance, à partir de la zone élémentaire d'illumination 18, à laquelle émanent les photons rétrodiffusés. Cela correspond à la distance entre la zone élémentaire d'illumination 18 et une zone élémentaire de détection 28n.

Lorsque son facteur de grandissement est supérieur à 1, le système optique 30 tend à rapprocher significativement les zones élémentaires de détection 28n de la zone élémentaire d'illumination 18 par rapport à la configuration de l'art antérieur, dépourvue de système optique, et représentée sur la figure 3B dans laquelle les extrémités distales de chaque fibre optique (fibres optiques de détection et fibre optique d'illumination) sont placées au contact de la surface de l'échantillon. Dans cette dernière configuration, la distance de rétrodiffusion Dn est égale à la distance dn entre les extrémités distales respectives d'une fibre optique de détection 22n et de la fibre optique d'illumination 12.

En résumé, dans la configuration de l'art antérieur, $Dn = dn$, tandis qu'en mettant en œuvre le système optique 30, $Dn = \frac{dn}{G}$. G

Si la distance entre le faisceau lumineux et l'extrémité distale d'une fibre optique de détection est, en amont du système optique, égale à une première distance, la distance de rétrodiffusion est à la surface de l'échantillon, égale à ladite première distance pondérée par l'inverse dudit facteur de grandissement.

Le terme « en amont » se comprend en considérant la direction de propagation de la lumière. Lorsque le facteur de grandissement G est inférieur à 1, le système optique tend au contraire à éloigner significativement les zones élémentaires de détection 28n de la zone élémentaire d'illumination 18 par rapport à la configuration de l'art antérieur.

Ainsi, selon la valeur du facteur de grandissement, les zones élémentaires de détection 28n sont :

- soit rapprochées les unes des autres, et rapprochées de la zone élémentaire d'illumination, leur surface étant également diminuée du carré du facteur de grandissement, par rapport à la surface de l'extrémité distale à laquelle elles sont respectivement associées. Cela permet d'effectuer une caractérisation de l'échantillon avec une résolution spatiale élevée, sur une profondeur plus faible que selon l'art antérieur.

- soit éloignées les unes des autres, et éloignées de la zone élémentaire d'illumination, leur surface étant augmentée du carré du facteur de grandissement, par rapport à la surface de l'extrémité distale à laquelle elles sont respectivement associées. Cela permet d'effectuer une caractérisation de l'échantillon avec une résolution spatiale plus faible, mais sur une profondeur plus élevée que selon l'art antérieur.

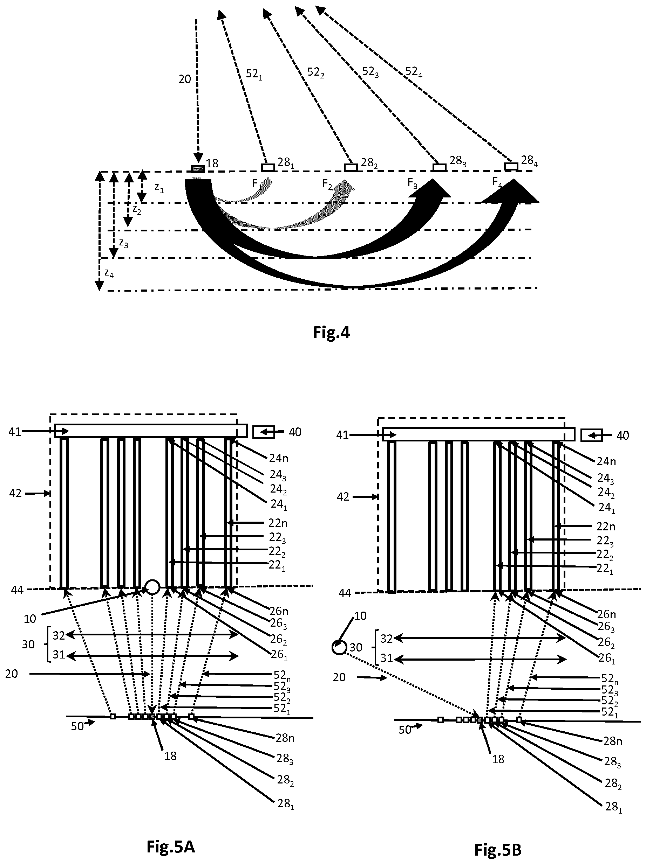

La figure 4 résume cet effet technique, chaque flèche $F_1$, $F_2$, $F_3$ et $F_4$ représentant le trajet, dans l'échantillon 50, des photons contribuant aux signaux rétrodiffusés respectifs $52_1$, $52_2$, $52_3$, $52_4$ respectivement associés aux zones élémentaires de détection $28_1$, $28_2$, $28_3$, $28_4$. Plus l'écartement entre les zones élémentaires de détection $28_1$, $28_2$, $28_3$, $28_4$ et la zone élémentaire d'illumination 18 est élevée, plus la profondeur examinée $z_1$, $z_2$, $z_3$, $z_4$ de l'échantillon est importante. Inversement, plus l'écartement entre les zones élémentaires de détection et la zone élémentaire d'illumination est faible plus la profondeur examinée de l'échantillon est faible.

Plus particulièrement, lorsque l'échantillon est de la peau, le fait de rapprocher les zones élémentaires de détection de la zone élémentaire d'illumination permet la caractérisation de l'épiderme, qui constitue la couche superficielle de la peau, s'étendant jusqu'à une profondeur comprise entre 100 et 300 $\mu$m, ce qui n'est pas envisageable avec les dispositifs de l'art antérieur, dont l'espacement entre chaque fibre est de l'ordre de quelques centaines de micromètres à quelques millimètres.

Autrement dit, le dispositif selon l'invention permet de détecter des signaux de rétrodiffusion émis par l'échantillon à des distances de rétrodiffusion significativement plus faibles que selon l'art antérieur, et cela permet une caractérisation des propriétés optiques de la couche superficielle de l'échantillon observé.

Lorsque le photodétecteur est un spectromètre, le dispositif permet d'effectuer des mesures du spectre rétrodiffusé selon des distances de rétrodiffusion de quelques dizaines à environ 200 $\mu$m. On parle de spectroscopie de microréflectance diffuse, par opposition à la spectroscopie de réflectance diffuse, connue de l'art antérieur.

De façon alternative, le dispositif peut ne pas comprendre de fibre optique d'illumination 22, sous réserve que la source de lumière 10 soit apte à produire un faisceau lumineux 20 vers la surface de l'échantillon 50, de façon à former, à cette surface, une zone élémentaire d'illumination 18 telle que précédemment décrite. Ce cas de figure est représenté sur la figure 5A, dans laquelle la source de lumière est disposée dans le plan de détection 44.

Selon une variante, également représentée sur la figure 5B, la source de lumière 10 n'est pas couplée au système optique.

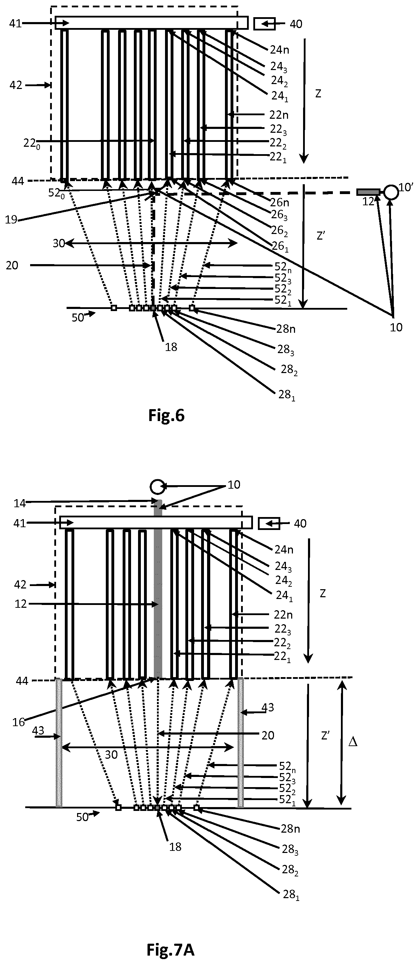

La figure 6 représente un autre mode de réalisation, reprenant les mêmes éléments que ceux décrits en lien avec la figure 1, à l'exception de la fibre optique d'illumination 12. En effet, dans ce mode de réalisation, cette dernière ne s'étend pas parallèlement à l'axe optique Z' du système optique 30, mais perpendiculairement à ce dernier. Une lame semi-réfléchissante 19, faisant office de renvoi d'angle, réfléchit le faisceau lumineux, issu de l'extrémité distale 16 de la fibre d'illumination 12, en direction de l'échantillon 50. Aussi, cette lame semi-réfléchissante 19 fait partie de la source de lumière 10 au sens de l'invention.

L'intérêt de cette configuration est qu'elle permet de collecter un rayonnement optique réfléchi à 180° par la surface de l'échantillon, ainsi qu'un rayonnement optique rétrodiffusé sous un angle de 180° ou proche de 180°, à quelques degrés près. Un tel rayonnement se dirige parallèlement au faisceau lumineux 20, puis est transmis par la lame semi-réfléchissante 19 vers une fibre optique de détection $22_0$, coaxiale dudit faisceau lumineux 20, cette fibre optique étant conjuguée à la zone élémentaire d'illumination 18 formée par la trace du faisceau d'illumination 20 sur la surface de l'échantillon.

Le rayonnement réfléchi peut comporter une information intéressante quant à l'état de surface de l'échantillon, notamment la brillance ou la couleur. Le rayonnement rétrodiffusé selon une plage angulaire 180° $\pm$ ε, avec ε $\leq$ 5°, voire ε $\leq$ 3° est porteur d'une information de diffusion sur couche de l'échantillon dite surfacique, s'étendant entre la surface de l'échantillon et une profondeur de 10 à 20 μm en dessous de cette dernière.

Alternativement, la fibre optique d'illumination 12 peut être remplacée par une source de lumière 10, sous réserve que cette dernière soit suffisamment ponctuelle.

La figure 7A représente un autre mode de réalisation, reprenant les mêmes éléments que ceux décrits en liaison avec le premier mode de réalisation. Selon ce mode de réalisation, le dispositif comporte un support 43, s'étendant selon l'axe optique Z' du système optique 30, apte à être appliqué contre l'échantillon 50. Cela permet une maîtrise de la position du système optique par rapport à l'échantillon analysé, et en particulier en s'assurant que la distance Δ entre le système optique et la surface de l'échantillon correspond à une distance de consigne, correspondant à un couplage optique optimal entre la surface de l'échantillon et les extrémités proximales des fibres optiques de détection. Ce couplage optimal est atteint lorsque la surface de l'échantillon examiné se trouve au foyer du système optique 30.

Le support peut notamment être solidaire de l'élément de maintien 42.

Selon des variantes de ce mode de réalisation, d'autres moyens pour contrôler cette distance Δ peuvent être considérés, en particulier en utilisant le fait que l'intensité du signal lumineux collecté par une fibre de détection est maximale lorsque la distance Δ correspond à la distance de consigne. Ces autres moyens de contrôle peuvent être par exemple :

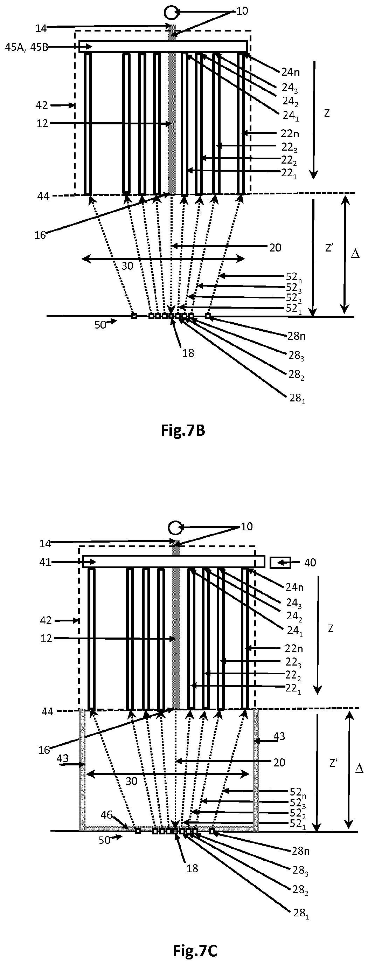

- une caméra 45A, représentée sur la figure 7B focalisée sur au moins l'extrémité proximale 24 d'une fibre optique de détection 22 : lorsque le système optique 30 est à la distance de consigne Δ de l'échantillon, l'image de l'extrémité distale 24, obtenue sur la caméra est une zone circulaire d'intensité maximale.

- un photocapteur 45B, représenté sur la figure 7B, de type photodiode, non résolu spatialement, apte à mesurer l'intensité du signal optique émergeant de l'extrémité distale 24 considérée : lorsque cette intensité est maximale, la distance Δ correspond à la valeur de consigne.

Le recours à un système optique de contrôle de la distance permet une détermination expérimentale de la distance de consigne Δ, en analysant l'évolution du signal mesuré en fonction de la distance entre le système optique 30 et l'échantillon 50.

Selon une variante représentée sur la figure 7C, le dispositif peut comporter une fenêtre transparente 46, s'étendant perpendiculairement à l'axe optique Z', de façon à venir s'appuyer sur la surface de l'échantillon. L'application d'une telle fenêtre 46 contre la surface de l'échantillon permet de faire en sorte que la surface examinée épouse la forme de la fenêtre et soit de ce fait maîtrisée. De préférence, la fenêtre transparente est plane. La fenêtre peut être obtenue en utilisant un matériau transparent de type polycarbonate, plexiglas, verre, l'épaisseur étant typiquement comprise entre 300 μm et quelques millimètres. L'expérience montre qu'en utilisant cette fenêtre sur des échantillons tels que la peau ou un autre tissu biologique, les mesures du signal optique rétrodiffusé 52 sont plus reproductibles.

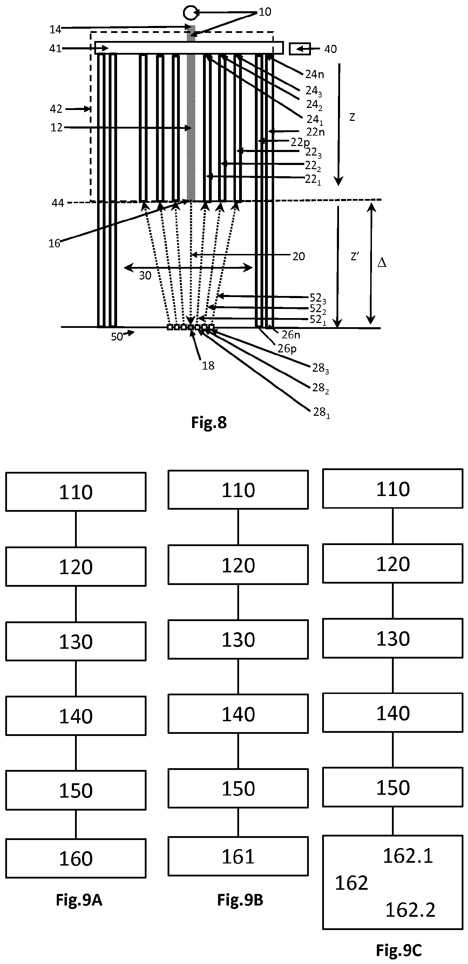

La figure 8 représente un autre mode de réalisation, reprenant les mêmes éléments que ceux décrits en liaison avec les modes de réalisation précédents. Selon ce mode de réalisation, le dispositif comporte au moins une fibre de détection 22n, 22p s'étendant jusqu'à la surface de l'échantillon, en étant, de préférence, parallèle à l'axe optique Z' du système optique 30. Ce dispositif permet de combiner :

- des mesures de spectroscopie de microréflectance diffuse, en mettant en œuvre les fibres de détection conjuguées à des zones de détection ($28_1$, $28_2$) placées à des distances micrométriques de la zone élémentaire d'illumination 18, le terme micrométrique désignant une distance inférieure à 1mm. On caractérise alors la couche superficielle de l'échantillon, qui s'étend selon une profondeur de 100 à 300 μm par rapport à la surface.
- des mesures de spectroscopie de réflectance diffuse, de façon analogue à l'art antérieur, en mettant en œuvre les fibres optiques de détection 22n, 22p s'étendant jusqu'à la surface de l'échantillon 50, et distantes de quelques millimètres de la zone élémentaire d'illumination. L'extrémité distale 26n, 26p de ces fibres est alors placée au contact de l'échantillon 50. On caractérise alors une couche plus profonde de l'échantillon, qui s'étend par exemple au-delà d'une profondeur supérieure à 300 μm par rapport à la surface.

On comprend que ce dispositif peut caractériser à la fois la couche superficielle et la couche profonde d'un échantillon.

On décrit à présent un procédé susceptible d'être mis en œuvre par les dispositifs précédemment décrits, afin d'estimer une ou plusieurs propriétés optiques de l'échantillon examiné.

Le terme propriété optique peut désigner :

- un facteur gouvernant l'absorption et la diffusion des photons dans le milieu diffusant, en particulier un coefficient d'absorption, un coefficient de diffusion, un coefficient de diffusion réduit, un coefficient d'anisotropie de la diffusion,

- une propriété d'émission d'un signal de fluorescence sous l'effet d'un faisceau incident, la fluorescence pouvant être endogène ou exogène,

- un décalage fréquentiel entre un faisceau incident et un faisceau rétrodiffusé par l'échantillon, mis à profit dans le cadre d'analyses de type spectrométrie Raman.

En lien avec la figure 9A, on décrit les principales étapes d'un procédé permettant l'estimation des propriétés optiques de diffusion, en particulier le coefficient d'absorption $\mu_a$ et le coefficient de diffusion réduit $\mu'_s$. Dans cet exemple, le facteur de grandissement G s'élève à 6. Comme représenté sur la figure 2, le dispositif comporte 36 fibres de détection, divisées en 6 groupes de 6 fibres, en fonction de la distance $d_n$ séparant chaque fibre de détection 22n du faisceau d'illumination 50. Ces distances $d_n$ s'élèvent respectivement à 300 $\mu$m ; 700 $\mu$m ; 1.1 mm ; 1.5 mm ; 2 mm ; 2.5 mm.

Grâce au système optique 30, chaque fibre de détection 22n est conjuguée à une zone élémentaire de détection 28n. Les zones élémentaires de détection sont alors elles-mêmes divisées en 7 groupes, en fonction de la distance de rétrodiffusion $D_n$ séparant chaque zone élémentaire de détection 28n à la zone élémentaire d'illumination 18. Compte tenu du facteur de grandissement, les distances $D_n$ s'élèvent respectivement à environ 50 $\mu$m ; 120 $\mu$m ; 185 $\mu$m ; 250 $\mu$m ; 333 $\mu$m ; 415 $\mu$m.

On procède alors selon les étapes suivantes :

1ère étape 110 : application du dispositif préalablement décrit, en regard de l'échantillon 50, de telle sorte que la surface examinée soit placée au foyer du système optique 30.

2ième étape 120 : illumination de l'échantillon en dirigeant un faisceau lumineux 20 contre la surface de l'échantillon, la partie de la surface illuminée constituant la zone élémentaire illuminée 18.

3ième étape 130 : collection d'un signal optique $52_1$, $52_2$, $52_3$, $52_4$... $52_n$ respectivement rétrodiffusé par l'échantillon, au niveau de chaque zone élémentaire de détection $28_1$, $28_2$, $28_3$, $28_4$... $28_n$, par la fibre optique $22_1$, $22_2$, $22_3$, $22_4$... $22_n$ de détection dont l'extrémité distale $26_1$, $26_2$, $26_3$, $26_4$... $26_n$ est respectivement conjuguée à ladite zone élémentaire $28_1$, $28_2$, $28_3$, $28_4$... $28_n$.

4ième étape 140 : mesure, à l'aide d'un photodétecteur 40, du signal $S_n$ représentatif de chaque signal optique rétrodiffusé $52_n$ à chaque distance $D_n$ de la zone élémentaire d'illumination. Le signal $S_n$ peut être notamment établi en cumulant les signaux optiques collectés par les fibres optiques de détection d'un même groupe. Lorsque le détecteur est un détecteur spectrométrique, ce qui constitue le mode de réalisation préféré, il génère le spectre du signal mesuré $S_n$.

5ième étape 150 : A l'aide de chaque signal $S_n$ correspondant à une distance de rétrodiffusion $D_n$, détermination d'une fonction $R_n$ appelée réflectance du signal, cette réflectance étant fonction du signal $S_n$ et de paramètres de calibration. Ainsi, $R_n = f_{calib}(S_n)$, ou $f_{calib}$ désigne une fonction de calibration, dépendant de l'instrumentation mise en œuvre, par exemple l'efficacité de collection par les fibres, la fonction de réponse du détecteur et l'intensité du faisceau lumineux incident. La fonction de calibration $f_{calib}$ peut être obtenue au cours d'une phase de calibration, préalablement ou postérieurement à la mesure sur l'échantillon.

Par exemple, la réflectance $R_n$ peut être obtenue, à partir de $S_n$, selon l'expression :

$$R_n = f_{calib}(S_n) = \frac{S_n - S_{ref}}{S_{source}}$$

ou selon l'expression

$$R_n = f_{calib}(S_n) = \frac{S_n - S_{ref}}{S_{source}} \times \frac{R_{std-n}}{S_{std-n}}$$

où :

- $S_n$ est le signal détecté correspondant à la distance $D_n$ de la zone élémentaire d'illumination.
- $S_{ref}$ est un signal de calibration, traduisant les réflexions parasites du système optique, obtenu en activant la source de lumière, mais sans présence de l'échantillon, ce dernier étant par exemple remplacé par un écran absorbant de type écran noir.
- $S_{source}$ est le signal produit par la source de lumière. Ssource peut notamment être établi en couplant la source de lumière au photodétecteur, par exemple au moyen d'une fibre optique dite de retour d'excitation 13, représentée sur la figure 1.
- $S_{std-n}$ est un signal de rétrodiffusion mesuré, en considérant une distance de rétrodiffusion $D_n$, sur un fantôme, dont les propriétés optiques (absorption, diffusion) sont connues, en utilisant le même dispositif que celui mis en œuvre pour acquérir le signal Sn.
- $R_{std-n}$ est une estimation de la réflectance produite, par ledit fantôme, à la distance de rétrodiffusion $D_n$.

D'une façon générale, la réflectance représente l'intensité du signal rétrodiffusé. Elle est généralement normalisée par l'intensité du faisceau incident au détecteur, auquel cas elle représente une fraction du faisceau incident rétrodiffusée.

6ième étape 160 : pour au moins une longueur d'onde $\lambda$, et en considérant au moins deux distances $D_n$ de rétrodiffusion, détermination du couple $(\mu_a(\lambda), \mu'_s(\lambda))$ présentant le moindre écart entre la réflectance mesurée $R_n(\lambda)$, à la longueur d'onde $\lambda$, et une réflectance modélisée $R_{n,\mu_a,\mu_s}^{model}(\lambda)$ pour différentes valeurs de $\mu_a(\lambda)$ et de $\mu'_s(\lambda)$, à une distance de rétrodiffusion $D_n$. Cette détermination peut être réalisée par la minimisation d'un écart quadratique, et par exemple selon l'expression :

$$\left(\mu_a(\lambda), \mu'_s(\lambda)\right) = argmin_{\left(\mu_a(\lambda),\mu'_s(\lambda)\right)} \left(\sum_{n=1}^{N} \left(R_{n,\mu a,\mu s'}^{model}(\lambda) - R_n(\lambda)\right)^2\right)$$

où :

- N désigne le nombre de distances prises en compte,
- $R_{n,\mu a,\mu s'}^{model}$ est une fonction de réflectance modélisée, établie pour la distance de rétrodiffusion $D_n$, et pour un couple de valeurs $\mu_a$ et $\mu_s$'.

Les différentes valeurs de réflectance modélisée $R_{n,\mu a,\mu s'}^{model}$ sont obtenues, pour une pluralité de couples de valeurs $\mu_a$, $\mu_s$' au cours d'une phase de calibration, par simulation numérique, ou expérimentalement, sur des échantillons étalons dont les propriétés optiques sont connues.

D'une façon générale, la notation $R_{n,p}^{model}$, désigne une réflectance modélisée à la distance de rétrodiffusion $D_n$, en prenant en compte des valeurs prédéterminées d'au moins un paramètre optique p. Le paramètre p peut correspondre à une propriété optique, ou un ensemble de propriétés optiques.

Les étapes 150 et 160 sont mises en œuvre par le microprocesseur 48, préalablement programmé à cet effet, et dont les données d'entrée sont les mesures réalisées par le photodétecteur 40.

Les inventeurs ont constaté que lorsque la distance de rétrodiffusion, séparant la zone d'illumination élémentaire d'une zone de détection élémentaire, est inférieure à 200 $\mu$m, ou, plus généralement, inférieure à un ratio 1/$\mu_s$', le procédé décrit ci-dessus permet de caractériser les propriétés optiques de la couche superficielle de l'échantillon examiné, cette couche superficielle s'étendant entre la surface de l'échantillon et une profondeur inférieure à 1/$\mu_s$'. Lorsque l'échantillon est de la peau d'un animal ou d'un être humain, une telle configuration permet une caractérisation des propriétés optiques de l'épiderme.

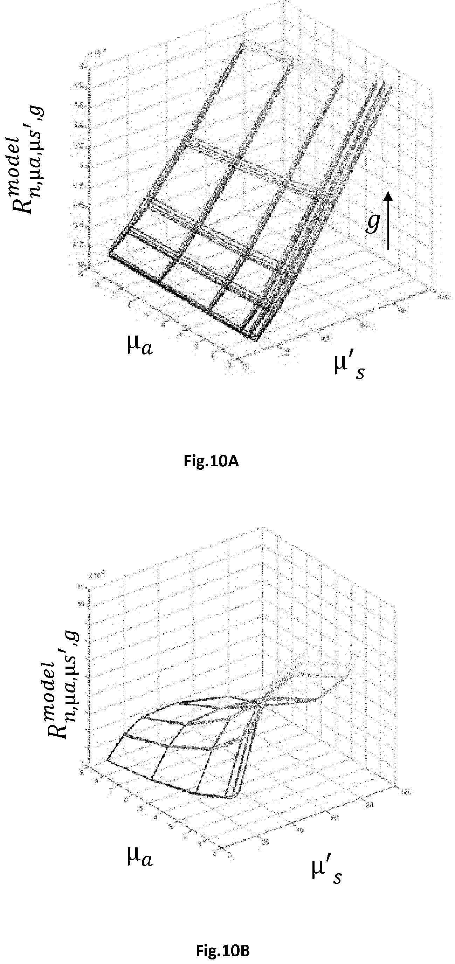

Les figures 10A et 10B, réalisées par les inventeurs, représentent la valeur de la réflectance R en fonction des coefficients $\mu$a , $\mu$s' et g, en considérant un milieu semi-infini. Ces simulations ont été réalisées avec un code de calcul modélisant le transport des photons de type Monte Carlo. La distance de rétrodiffusion est respectivement égale à 50 $\mu$m pour la figure 10A et 415 $\mu$m pour la figure 10B. Sur chaque figure, on a considéré 4 valeurs de g : 0,6 ; 0,7 ; 0,8 ; 0, 9.

Le facteur d'anisotropie de la diffusion, noté g, représente le cosinus moyen de l'angle de diffusion au cours d'une diffusion. Ce facteur, sans dimension, est défini dans la littérature par l'expression :

$$g = \int_{-1}^{1} f(\cos(\theta))\cos(\theta)d(\cos(\theta))$$

où

- $\theta$ représente l'angle de diffusion,

- $f(\cos(\theta))$ est la probabilité associée à chaque valeur du cosinus de l'angle de diffusion.

Une diffusion parfaitement anisotrope, dans laquelle tous les angles de diffusion seraient équiprobables, se traduit par un coefficient d'anisotropie égal à 0. A l'inverse, en l'absence de diffusion, les photons ne sont pas déviés et le coefficient d'anisotropie est égal à 1. La propagation de la lumière dans le milieu est alors uniquement gouvernée par le coefficient d'absorption.

Dans les milieux biologiques, il est admis que la diffusion est anisotrope, privilégiant les faibles angles de diffusion. On parle de diffusion vers l'avant. Le facteur d'anisotropie est choisi de façon arbitraire, et généralement entre 0,8 et 0,95. Ce facteur est alors utilisé en tant que facteur de correction appliqué au coefficient de diffusion $\mu_s$, afin d'établir un coefficient de diffusion dit réduit, $\mu_s'$, tel que :

$$\mu_s' = \mu_s (1-g)$$

$\mu_s'$ est un coefficient de diffusion réduit, prenant en compte la nature anisotrope de la diffusion dans le milieu diffusant.

Sur la base de la figure 10B, qui correspond à une distance de rétrodiffusion supérieure à quelques centaines de $\mu$m, l'influence de la valeur du coefficient d'anisotropie g sur la réflectance est faible : quelle que soit sa valeur, la valeur de la réflectance, pour un couple ($\mu$a, $\mu$s') donné, n'évolue pas de façon significative. La diffusion de la lumière est correctement décrite par les coefficients $\mu$a et $\mu$s' moyennant une détermination arbitraire du coefficient d'anisotropie.

En revanche, lorsque la distance de rétrodiffusion est faible, par exemple inférieure à 100 $\mu$m, comme c'est le cas sur la figure 10A, ce qui est rendu possible par le dispositif mis en œuvre, la réflectance évolue significativement en fonction du coefficient d'anisotropie. Sur cette figure, les différentes nappes, correspondant à différentes valeurs de g, se séparent. Aussi, une même valeur de réflectance peut correspondre à plusieurs couples ($\mu$a, $\mu$s'), selon la valeur du coefficient d'anisotropie g. Ainsi, la prise en compte d'un coefficient d'anisotropie défini de façon arbitraire n'est plus adaptée. Il devient alors utile de le déterminer expérimentalement.

Autrement dit, au voisinage de la surface de l'échantillon, la diffusion des photons ne peut plus être modélisée, de façon satisfaisante, en considérant le uniquement coefficient d'absorption $\mu_a$ et le coefficient de diffusion réduit $\mu_s'$, mais en considérant également le coefficient d'anisotropie de la diffusion.

Comme représenté sur la figure 9B, et sous réserve de disposer d'un nombre suffisant de mesures à des distances de rétrodiffusion $D_n$ différentes et inférieures à 200 $\mu$m, on peut déterminer conjointement $\mu$a, $\mu$s' et g, (ou $\mu$a, $\mu$s et g) en appliquant les étapes 110 à 150 préalablement décrites, l'étape 160 étant remplacée par une étape 161 dans laquelle, pour au moins une longueur d'onde $\lambda$, et pour une pluralité de distances Dn, inférieures à 200 $\mu$m, on détermine le triplet ($\mu_a(\lambda)$, $\mu'_s(\lambda)$ ,g) présentant le moindre écart entre la réflectance $R_n(\lambda)$ mesurée, à la longueur d'onde $\lambda$, et une réflectance modélisée $R_{n,\mu a,\mu s',g}^{model}$ pour différentes valeurs du triplet ($\mu$a, $\mu$s', g). Cette détermination peut être réalisée en minimisant un écart quadratique, selon l'expression :

$$\left(\mu_a(\lambda), \mu'_s(\lambda), g(\lambda)\right) = argmin_{\mu a,\mu s',g}\left(\sum_{n=1}^{N}\left(R_{n,\mu a,\mu s',g}^{model}(\lambda,) - R_n(\lambda)\right)^2\right)$$

où :

- N désigne le nombre de distances prises en compte. Dans le cas présent, N doit être au moins supérieur à 3 car trois grandeurs sont à estimer, à savoir $\mu_a$, $\mu_s'$ et g.

- $R_{n,\mu a,\mu s',g}^{model}$ est une fonction de réflectance modélisée, établie pour une distance de rétrodiffusion $D_n$, et pour différents triplets de valeurs $\mu_a$, $\mu s'$ et g, au cours d'une phase de calibration, comme précédemment décrit. De telles valeurs aboutissent à des tables, dites Look Up Tables, telles que représentées sur les figures 10A et 10B.

Cette étape 161 peut être avantageusement mise en œuvre itérativement, en fixant, par exemple alternativement, deux paramètres, sur les trois recherchés, soit à une valeur initiale arbitraire soit à une valeur déterminée lors d'une itération précédente. L'algorithme itératif peut alors converger vers un triplet de valeurs $(\mu_a, \mu_s', g)$.

Les inventeurs ont par ailleurs réalisé différents modèles, montrant que lorsque la distance de rétrodiffusion est faible, typiquement inférieure à $1 / \mu_s'$, l'influence du coefficient d'absorption $\mu_a$ sur la réflectance est faible.

Tirant profit de ces constatations, il est proposé un procédé préférentiel de détermination des propriétés optiques de diffusion, plus robuste que le procédé précédent, et représenté sur la figure 9C, comprenant les étapes 110 à 150 telles que précédemment décrites, l'étape 120 comprenant l'acquisition :

- de deux signaux optiques de rétrodiffusion ($52_1$, $52_2$), dits signaux proches, à deux distances de rétrodiffusion $D_1$ et $D_2$ dites « proches », inférieures à 200 $\mu$m, ou, de façon plus générale, inférieures à $1/\mu s'$.

- de deux signaux optiques de rétrodiffusion, dits signaux éloignés, ($52_3$, $52_4$) à deux distances de rétrodiffusion $D_3$ et $D_4$ dites « éloignées », supérieures aux dites distances proches ($D_1$, $D_2$) et de préférence supérieures à 200 $\mu$m, ou, de façon plus générale, supérieures à $1/\mu s'$, et de préférence supérieures à 500 $\mu$m.

L'étape 160 est remplacée par une étape 162, comprenant :

- Sous-Etape 162.1 : la détermination, pour au moins une longueur d'onde $\lambda$, ou pour au moins une bande spectrale, du coefficient de diffusion réduit $\mu_s'(\lambda)$ et du coefficient d'absorption $\mu_a(\lambda)$ par comparaison des réflectances $R_3$ et $R_4$, mesurées aux distances de rétrodiffusion éloignées $D_3$ et $D_4$, avec des valeurs de réflectances modélisées $R_{3,\mu a,\mu s'}^{model}$, $R_{4,\mu a,\mu s'}^{model}$ de façon analogue à l'étape 160 précédemment décrite. Cette comparaison peut prendre la forme d'une minimisation d'un écart quadratique, selon l'expression :

$$\left(\mu_a(\lambda), \mu'_s(\lambda)\right) = argmin_{\left(\mu_a(\lambda),\mu'_s(\lambda)\right)} \left( \sum_{n=3}^{n=4} \left( R_{n,\mu a,\mu s'}^{model}(\lambda) - R_n(\lambda) \right)^2 \right)$$

- Sous-étape 162.2 : la détermination, pour la même longueur d'onde, ou la même bande spectrale, du coefficient de diffusion $\mu_s(\lambda)$ et du coefficient d'anisotropie g, par comparaison des réflectances $R_1$ et $R_2$, mesurées aux distances courtes $D_1$ et $D_2$, avec des valeurs de réflectances $R_1^{model}$ et $R_2^{model}$, modélisées en considérant respectivement les mêmes distances de rétrodiffusion et différentes valeurs de $\mu_s$ et de g, de façon analogue à l'étape 160 précédemment décrite. Cette comparaison peut prendre la forme d'une minimisation d'un écart quadratique, selon l'expression :

$$\left(\mu_s(\lambda), g(\lambda)\right) = argmin_{(\mu s(\lambda),g)} \left( \sum_{n=1}^{n=2} \left( R_{n,\mu s,g}^{model}(\lambda) - R_n(\lambda) \right)^2 \right)$$

ou $R_{n,\mu s,g}^{model}$ désigne une réflectance modélisée à la distance n, et un couple de valeurs $(\mu_s, g)$.

Cet algorithme est mis en œuvre en considérant la contrainte suivante :

$$\mu_s'(\lambda) = \mu_s(\lambda) \, (1-g(\lambda))$$

$\mu_s'(\lambda)$ étant déterminé lors de la première étape 162.1.

Par ailleurs, le fait de déterminer $\mu_a$, lors de la première étape, permet, lors de la deuxième étape de sélectionner des valeurs de la réflectance modélisée $R_{n,\mu s,g}^{model}$ tenant compte de la valeur $\mu_a$ ainsi établie. On voit tout l'intérêt de combiner des mesures à grandes et à faibles distance de rétrodiffusion.

Lorsque la valeur du coefficient d'absorption est connue, ou fixée arbitrairement, l'étape 162.1 vise uniquement à déterminer $\mu_s'(\lambda)$ en prenant en compte la valeur dudit coefficient d'absorption. Dans ce cas, il n'est pas nécessaire de disposer de 2 mesures $D_3$, $D_4$ à des distances éloignées. Une mesure à une seule distance ($D_3$) peut suffire.

Lorsque le détecteur est pourvu d'une fonction spectrométrique, les procédés précédemment décrits peuvent être mises en œuvre pour une pluralité de longueurs d'onde ou de bandes spectrales différentes. Dans ce cas, on obtient les différentes propriétés optiques en fonction de la longueur d'onde ou de la bande spectrale considérée.

Dans les exemples décrits ci-dessus, le dispositif met en œuvre un photodétecteur de type spectrophotomètre, mais d'autres types de photodétecteurs sont utilisables, par exemple un photodétecteur apte à réaliser un spectre Raman, ou un photodétecteur non spectralement résolu, par exemple une photodiode, de préférence sensible à une bande spectrale étroite, ou tout autre photodétecteur permettant la détection d'un signal de fluorescence.

Dans ce dernier cas, le photodétecteur peut notamment être un photodétecteur résolu en temps, la source de lumière étant une source impulsionnelle. La propriété optique à déterminer est alors la position d'un fluorophore ou d'un absorbeur dans l'échantillon diffusant examiné, ce dernier étant décomposé en voxels. Des algorithmes adaptés à la localisation de fluorophores ou d'absorbeurs, adaptés à des configurations en rétrodiffusion, ont été décrits par la demanderesse dans les documents US8253116B2, US8193518B2, ou US8263947B2. Ces procédés de reconstruction, faisant partie de l'art antérieur, nécessitent la prise en compte paramètres de diffusion optique dans l'échantillon, notamment le coefficient de diffusion réduit ou le coefficient de diffusion. Ces propriétés optiques peuvent être obtenues par un des procédés décrits en lien avec les figures 9A à 9C.

**Revendications**

1. Dispositif de mesure d'un signal optique produit par un échantillon comportant :

    - une source de lumière (10) apte à émettre un faisceau lumineux (20) vers une surface dudit échantillon (50), de façon à former, sur ladite surface, une zone élémentaire d'illumination (18),
    - au moins une fibre optique de détection (22), s'étendant entre une extrémité proximale (24), apte à être couplée à un photodétecteur (40), et une extrémité distale (26), apte à collecter un signal optique (52) rétrodiffusé par ledit échantillon lorsqu'il est exposé audit faisceau lumineux,
    - un système optique (30), présentant un facteur de grandissement (G) et un axe optique (Z'),
    - ledit système optique (30) étant apte à conjuguer l'extrémité distale de chaque fibre optique de détection (26) à une zone élémentaire de détection (28, 28n) située sur la surface de l'échantillon, de telle sorte que la distance (Dn), dite distance de rétrodiffusion, entre la zone élémentaire d'illumination (18) et chaque zone élémentaire de détection (28, 28n), perpendiculairement audit axe optique, est fonction dudit facteur de grandissement,
    le dispositif étant **caractérisé en ce que** le système optique (30) est configuré de telle sorte qu'au moins une zone élémentaire de détection est séparée de ladite zone élémentaire d'illumination (18), la distance de rétrodiffusion séparant ladite zone élémentaire de détection de ladite zone élémentaire d'illumination étant inférieure à 200 $\mu$m.

2. Dispositif selon la revendication 1, comportant plusieurs fibres optiques de détection de manière à définir plusieurs distances de rétodiffusion différentes et inférieures à 200 $\mu$m.

3. Dispositif selon l'une quelconque des revendications précédentes, comportant :

    - une première fibre optique de détection ($22_1$), dont l'extrémité distale est située à une première distance ($d_1$) dudit faisceau lumineux (20),
    - une deuxième fibre optique de détection ($22_2$), dont l'extrémité distale est située à une deuxième distance ($d_2$) dudit faisceau lumineux (20), ladite deuxième distance étant supérieure à ladite première distance.

4. Dispositif selon l'une quelconque des revendications précédentes, comportant :

    - un premier groupe ($G_1$) de premières fibres optiques de détection, dont l'extrémité distale est située à une

première distance ($d_1$) dudit faisceau lumineux (20),
- un deuxième groupe ($G_2$) de deuxièmes fibres optiques de détection, dont l'extrémité distale est située à une deuxième distance ($d_2$) dudit faisceau lumineux (20), ladite deuxième distance ($d_2$) étant supérieure à ladite première distance.

5. Dispositif selon la revendication 4, dans lequel :

- les extrémités distales desdites premières fibres de détection sont coplanaires et réparties autour dudit faisceau lumineux, le long d'un cercle de rayon égal à ladite première distance (d1), centré sur ledit faisceau lumineux (20),
- les extrémités distales desdites deuxièmes fibres de détection sont coplanaires et réparties autour dudit faisceau lumineux (20), le long d'un cercle de rayon égal à la deuxième distance (d2), centré sur ledit faisceau lumineux (20).

6. Dispositif selon l'une quelconque des revendications précédentes, la source de lumière (10) comportant une fibre optique d'illumination (12), s'étendant entre

- une extrémité proximale (14), apte à recevoir ladite lumière,
- et une extrémité distale (16), apte à émettre un faisceau lumineux en direction de la surface de l'échantillon (50).

7. Dispositif selon l'une quelconque des revendications 1 à 5, dans lequel la source de lumière (10) comporte un système de renvoi (19), apte à réfléchir ledit faisceau lumineux (20) en direction de l'échantillon (50).

8. Dispositif selon la revendication 7, dans lequel le système de renvoi est une lame semi-réfléchissante (19), le dispositif comportant une fibre de détection ($22_0$) apte à collecter un signal optique réfléchi ou rétrodiffusé à 180° par l'échantillon.

9. Dispositif selon l'une quelconque des revendications précédentes dans lequel la source de lumière (10) est choisie parmi une source de lumière blanche, ou une source Laser, ou une diode électroluminescente.

10. Dispositif selon l'une quelconque des revendications précédentes dans lequel ladite source de lumière (10) est apte à diriger ledit faisceau lumineux (20) parallèlement audit axe optique (Z').

11. Dispositif selon l'une quelconque des revendications précédentes, comportant un contrôleur de la distance (43) entre ledit système optique (30) et ladite surface de l'échantillon (50).

12. Dispositif selon la revendication 11, dans lequel ledit contrôleur de distance comporte :

- un analyseur de l'intensité lumineuse (45B) du signal optique collecté par une fibre optique de détection (22),
- ou une caméra (45A) couplée à l'extrémité proximale (24) d'au moins une fibre optique de détection (22).

13. Dispositif selon la revendication 11, dans lequel le contrôleur de distance comporte un support (43), s'étendant selon l'axe optique du système optique, et apte à être appliqué contre la surface de l'échantillon.

14. Dispositif selon la revendication 13, comportant une fenêtre transparente (46), solidaire dudit support, s'étendant perpendiculairement audit axe optique, la fenêtre étant apte à être appliquée contre la surface de l'échantillon (50), de telle sorte que cette dernière épouse la forme de la fenêtre.

15. Dispositif selon l'une quelconque des revendications précédentes, comportant un photodétecteur (40) choisi parmi une photodiode, un spectrophotomètre, un spectromètre Raman ou un détecteur d'un signal fluorescent.

16. Procédé de détermination d'une propriété optique, p, d'un échantillon, à l'aide d'un dispositif selon l'une quelconque des revendications 1 à 15, comportant les étapes suivantes :

- illumination d'une surface de l'échantillon (50) à l'aide d'un faisceau lumineux (20), de façon à constituer, à la surface dudit échantillon, une zone élémentaire d'illumination (18), correspondant à la partie de ladite surface éclairée par ledit faisceau,
- détection de N signaux optiques ($52_1$, $52_2$, $52_3$, $52_4$, $52_n$), rétrodiffusés par l'échantillon, chaque signal optique émanant de la surface de l'échantillon à une distance ($D_1$, $D_2$, $D_3$, $D_4$, $D_n$), dite distance de rétrodiffusion, de ladite zone élémentaire d'illumination, N étant un entier supérieur ou égal à 1, de manière à former des signaux détectés, $S_1$, $S_2$, $S_3$, $S_4$, $S_n$,
- détermination d'au moins une propriété optique, p, de l'échantillon, par comparaison entre :

- une fonction, $R_1$, $R_2$, $R_3$, $R_4$, $R_n$, de chaque signal ainsi détecté,
- et une pluralité d'estimations de ladite fonction, $R_{1,p}^{model}$, $R_{2,p}^{model}$, $R_{3,p}^{model}$, $R_{4,p}^{model}$, $R_{n,p}^{model}$, chaque estimation étant réalisée en considérant une valeur prédéterminée de ladite propriété optique, p.

## Patentansprüche

1. Vorrichtung zur Messung eines von einer Probe erzeugten optischen Signals, welche umfasst:

   - eine Lichtquelle (10), die dafür ausgelegt ist, einen Lichtstrahl (20) in Richtung einer Oberfläche der Probe (50) auszusenden, um auf der Oberfläche einen elementaren Beleuchtungsbereich (18) zu bilden,
   - wenigstens einen Detektions-Lichtwellenleiter (22), der sich zwischen einem proximalen Ende (24), das mit einem Photodetektor (40) koppelbar ist, und einem distalen Ende (26) erstreckt, das dafür ausgelegt ist, ein optisches Signal (52) zu sammeln, das von der Probe rückgestreut wird, wenn sie gegenüber dem Lichtstrahl exponiert ist,
   - ein optisches System (30), das einen Vergrößerungsfaktor (G) und eine optische Achse (Z') aufweist,
   - wobei das optische System (30) dafür ausgelegt ist, das distale Ende (26) jedes Detektions-Lichtwellenleiters mit einem elementaren Detektionsbereich (28, 28n) zu koppeln, der sich auf der Oberfläche der Probe befindet, derart, dass der Abstand (Dn), Rückstreuabstand genannt, zwischen dem elementaren Beleuchtungsbereich (18) und jedem elementaren Detektionsbereich (28, 28n), senkrecht zur optischen Achse, von dem Vergrößerungsfaktor abhängig ist,
   wobei die Vorrichtung **dadurch gekennzeichnet ist, dass** das optische System (30) derart gestaltet ist, dass wenigstens ein elementarer Detektionsbereich von dem elementaren Beleuchtungsbereich (18) getrennt ist, wobei der Rückstreuabstand, der den elementaren Detektionsbereich von dem elementaren Beleuchtungsbereich trennt, kleiner als 200 $\mu$m ist.

2. Vorrichtung nach Anspruch 1, welche mehrere Detektions-Lichtwellenleiter umfasst, um mehrere Rückstreuabstände zu definieren, die verschieden und kleiner als 200 $\mu$m sind.

3. Vorrichtung nach einem der vorhergehenden Ansprüche, welche umfasst:

   - einen ersten Detektions-Lichtwellenleiter ($22_1$), dessen distales Ende sich in einem ersten Abstand ($d_1$) von dem Lichtstrahl (20) befindet,
   - einen zweiten Detektions-Lichtwellenleiter ($22_2$), dessen distales Ende sich in einem zweiten Abstand ($d_2$) von dem Lichtstrahl (20) befindet, wobei der zweite Abstand größer als der erste Abstand ist.

4. Vorrichtung nach einem der vorhergehenden Ansprüche, welche umfasst:

   - eine erste Gruppe ($G_1$) von ersten Detektions-Lichtwellenleitern, deren distales Ende sich in einem ersten Abstand ($d_1$) von dem Lichtstrahl (20) befindet,
   - eine zweite Gruppe ($G_2$) von zweiten Detektions-Lichtwellenleitern, deren distales Ende sich in einem zweiten Abstand ($d_2$) von dem Lichtstrahl (20) befindet, wobei der zweite Abstand ($d_2$) größer als der erste Abstand ist.

5. Vorrichtung nach Anspruch 4, wobei:

   - die distalen Enden der ersten Detektions-Lichtwellenleiter koplanar und um den Lichtstrahl herum entlang eines Kreises verteilt sind, dessen Radius gleich dem ersten Abstand (d1) ist und der um den Lichtstrahl (20) zentriert ist,
   - die distalen Enden der zweiten Detektions-Lichtwellenleiter koplanar und um den Lichtstrahl (20) herum entlang eines Kreises verteilt sind, dessen Radius gleich dem zweiten Abstand (d2) ist und der um den Lichtstrahl (20) zentriert ist.

6. Vorrichtung nach einem der vorhergehenden Ansprüche, wobei die Lichtquelle (10) einen Beleuchtungs-Lichtwellenleiter (12) umfasst, der sich erstreckt zwischen:

- einem proximalen Ende (14), das dafür ausgelegt ist, das Licht zu empfangen,
- und einem distalen Ende (16), das dafür ausgelegt ist, einen Lichtstrahl in Richtung der Oberfläche der Probe (50) auszusenden.

7. Vorrichtung nach einem der Ansprüche 1 bis 5, wobei die Lichtquelle (10) ein Umlenksystem (19) umfasst, das dafür ausgelegt ist, den Lichtstrahl (20) in Richtung der Probe (50) zu reflektieren.

8. Vorrichtung nach Anspruch 7, wobei das Umlenksystem eine halbreflektierende Platte (19) ist, wobei die Vorrichtung einen Detektions-Lichtwellenleiter ($22_0$) umfasst, der dafür ausgelegt ist, ein optisches Signal zu sammeln, das von der Probe mit 180° reflektiert oder rückgestreut wird.

9. Vorrichtung nach einem der vorhergehenden Ansprüche, wobei die Lichtquelle (10) aus einer Weißlichtquelle oder einer Laserquelle oder einer Leuchtdiode ausgewählt ist.

10. Vorrichtung nach einem der vorhergehenden Ansprüche, wobei die Lichtquelle (10) dafür ausgelegt ist, den Lichtstrahl (20) parallel zur optischen Achse (Z') zu lenken.

11. Vorrichtung nach einem der vorhergehenden Ansprüche, welche eine Steuereinrichtung für den Abstand (43) zwischen dem optischem System (30) und der Oberfläche der Probe (50) umfasst.

12. Vorrichtung nach Anspruch 11, wobei die Steuereinrichtung für den Abstand umfasst:

- einen Analysator für die Lichtstärke (45B) des von einem Detektions-Lichtwellenleiter (22) gesammelten optischen Signals,
- oder eine Kamera (45A), die mit dem proximalen Ende (24) wenigstens eines Detektions-Lichtwellenleiters (22) gekoppelt ist.

13. Vorrichtung nach Anspruch 11, wobei die Steuereinrichtung für den Abstand einen Träger (43) umfasst, der sich entlang der optischen Achse des optischen Systems erstreckt und dafür ausgelegt ist, an die Oberfläche der Probe angedrückt zu werden.

14. Vorrichtung nach Anspruch 13, welche ein mit dem Träger fest verbundenes transparentes Fenster (46) umfasst, das sich senkrecht zur optischen Achse erstreckt, wobei das Fenster dafür ausgelegt ist, an die Oberfläche der Probe (50) angedrückt zu werden, derart, dass diese Letztere die Form des Fensters annimmt.

15. Vorrichtung nach einem der vorhergehenden Ansprüche, welche einen Photodetektor (40) umfasst, der aus einer Photodiode, einem Spektrophotometer, einem Raman-Spektrometer oder einem Detektor eines Fluoreszenzsignals ausgewählt ist.

16. Verfahren zur Bestimmung einer optischen Eigenschaft p einer Probe mithilfe einer Vorrichtung nach einem der Ansprüche 1 bis 15, welches die folgenden Schritte umfasst:

- Beleuchtung einer Oberfläche der Probe (50) mithilfe eines Lichtstrahls (20), um auf der Oberfläche der Probe einen elementaren Beleuchtungsbereich (18) zu bilden, welcher dem von dem Strahl beleuchteten Teil der Oberfläche entspricht,
- Detektion von N von der Probe rückgestreuten optischen Signalen ($52_1$, $52_2$, $52_3$, $52_4$, $52_n$), wobei jedes optische Signal von der Oberfläche der Probe in einem Abstand ($D_1$, $D_2$, $D_3$, $D_4$, $D_n$), Rückstreuabstand genannt, von dem elementaren Beleuchtungsbereich ausgeht, wobei N eine ganze Zahl größer oder gleich 1 ist, so dass detektierte Signale $S_1$, $S_2$, $S_3$, $S_4$, $S_n$ gebildet werden,
- Bestimmung wenigstens einer optischen Eigenschaft p der Probe durch Vergleich zwischen:

- einer Funktion $R_1$, $R_2$, $R_3$, $R_4$, $R_n$ jedes so detektierten Signals,
- und mehreren Schätzungen dieser Funktion, $R_{1,p}^{model}$, $R_{2,p}^{model}$, $R_{3,p}^{model}$, $R_{4,p}^{model}$, $R_{n,p}^{model}$, wobei jede Schätzung unter der Annahme eines vorbestimmten Wertes der optischen Eigenschaft p vorgenommen wird.

**Claims**

1. Device for measuring an optical signal produced by a sample, comprising:

   - a light source (10) able to emit a light beam (20) toward a surface of said sample (50), so as to form, on said surface, an elementary illumination region (18),
   - at least one detection optical fibre (22), extending between a near end (24), able to be coupled to a photodetector (40), and a far end (26), able to collect an optical signal (52) backscattered by said sample when it is exposed to said light beam,
   - an optical system (30), having a magnification factor (G) and an optical axis (Z'),
   - said optical system (30) being able to conjugate the far end of each detection optical fibre (26) with an elementary detection region (28, 28n) located on the surface of the sample, such that the distance (Dn), called the backscattering distance, between the elementary illumination region (18) and each elementary detection region (28, 28n), perpendicular to said optical axis, is dependent on said magnification factor,

   the device being **characterized in that** the optical system (30) is configured so that at least one elementary detection region is separated from said elementary illumination region (18), the backscattering distance separating said elementary detection region from said elementary illumination region being smaller than 200 $\mu$m.

2. Device according to Claim 1, comprising a plurality of detection optical fibres so as to define a plurality of different backscattering distances smaller than 200 $\mu$m.

3. Device according to any one of the preceding claims, comprising:

   - a first detection optical fibre ($22_1$), the far end of which is located at a first distance ($d_1$) from said light beam (20),
   - a second detection optical fibre ($22_2$), the far end of which is located at a second distance ($d_2$) from said light beam (20), said second distance being larger than said first distance.

4. Device according to any one of the preceding claims, comprising:

   - a first group ($G_1$) of first detection optical fibres, the far end of which is located at a first distance ($d_1$) from said light beam (20),
   - a second group ($G_2$) of second detection optical fibres, the far end of which is located at a second distance ($d_2$) from said light beam (20), said second distance ($d_2$) being larger than said first distance.

5. Device according to Claim 4, wherein:

   - the far ends of said first detection fibres are coplanar and distributed about said light beam, along a circle of radius equal to said first distance (d1), centred on said light beam (20),
   - the far ends of said second detection fibres are coplanar and distributed about said light beam (20), along a circle of radius equal to the second distance (d2), centred on said light beam (20).

6. Device according to any one of the preceding claims, the light source (10) comprising an illumination optical fibre (12), extending between
   a near end (14), able to receive said light,
   and a far end (16), able to emit a light beam in the direction of the surface of the sample (50).

7. Device according to any one of Claims 1 to 5, wherein the light source (10) comprises a steering system (19), able to reflect said light beam (20) in the direction of the sample (50).

8. Device according to Claim 7, wherein the steering system is a half-silvered mirror (19), the device comprising a detection fibre ($22_0$) able to collect an optical signal reflected or backscattered at 180° by the sample.

9. Device according to any one of the preceding claims, wherein the light source (10) is chosen from a white light source, a laser source, or a light-emitting diode.

10. Device according to any one of the preceding claims, wherein said light source (10) is able to direct said light beam (20) parallel to said optical axis (Z').

**11.** Device according to any one of the preceding claims, comprising a controller of the distance (43) between said optical system (30) and said surface of the sample (50).

**12.** Device according to Claim 11, wherein said distance controller comprises:

- an analyser of the light intensity (45B) of the optical signal collected by a detection optical fibre (22),
- or a camera (45A) coupled to the near end (24) of at least one detection optical fibre (22).

**13.** Device according to Claim 11, wherein the distance controller comprises a holder (43), extending along the optical axis of the optical system, and able to be applied against the surface of the sample.

**14.** Device according to Claim 13, comprising a transparent window (46), integrated into said holder, extending perpendicular to said optical axis, the window being able to be applied against the surface of the sample (50), so that the latter conforms to the shape of the window.

**15.** Device according to any one of the preceding claims, comprising a photodetector (40) chosen from a photodiode, a spectrophotometer, a Raman spectrometer or a detector of a fluorescent signal.

**16.** Method for determining an optical property, p, of a sample using a device according to any one of Claims 1 to 15, comprising the following steps:

- illuminating a surface of the sample (50) using a light beam (20), so as to form, on the surface of said sample, an elementary illumination region (18), corresponding to the portion of said surface illuminated by said beam,
- detecting N optical signals ($52_1$, $52_2$, $52_3$, $52_4$, $52_n$) backscattered by the sample, each optical signal emanating from the surface of the sample at a distance ($D_1$, $D_2$, $D_3$, $D_4$, $D_n$), called the backscattering distance, from said elementary illumination region, N being an integer greater than or equal to 1, so as to form detected signals $S_1$, $S_2$, $S_3$, $S_4$, $S_n$,
- determining at least one optical property, p, of the sample by comparison between:

a function, $R_1$, $R_2$, $R_3$, $R_4$, $R_n$, of each signal thus detected,
and a plurality of estimations of said function, $R_{1,p}^{model}$, $R_{2,p}^{model}$, $R_{3,p}^{model}$, $R_{4,p}^{model}$, $R_{n,p}^{model}$,
each estimation being carried out considering a preset value of said optical property, p.

**Fig.1**

**Fig.2**

Fig.3A

Fig.3B

**Fig.4**

**Fig.5A**              **Fig.5B**

**Fig.6**

**Fig.7A**

**Fig.7B**

**Fig.7C**

Fig.8

Fig.9A

Fig.9B

Fig.9C

$R^{model}_{n,\mu a,\mu s',g}$

$\mu_a$

$\mu'_s$

$g$

Fig.10A

$R^{model}_{n,\mu a,\mu s',g}$

$\mu_a$

$\mu'_s$

Fig.10B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010054203 A
- EP 1262763 A
- EP 1828753 A
- US 20110178379 A
- EP 2762064 A
- US 8253116 B2
- US 8193518 B2
- US 8263947 B2